# EUROPÄISCHE PATENTSCHRIFT

(11) **EP 0 651 734 B1**
(45) Veröffentlichungstag und Bekanntmachung des Hinweises auf die Patenterteilung: **01.05.1996**
(21) Anmeldenummer: 94910390.7
(22) Anmeldetag: 08.03.1994
(51) Int. Cl.: C07C 59/105, C07C 51/235, C07C 51/215

(54) **VERFAHREN UND VORRICHTUNG ZUR HERSTELLUNG VON MONOCARBONSÄUREN VON KOHLENHYDRATEN, KOHLENHYDRATDERIVATEN ODER PRIMÄREN ALKOHOLEN**
METHOD AND DEVICE FOR PRODUCING MONOCARBOXYLIC ACIDS FROM CARBOHYDRATES, CARBOHYDRATE DERIVATES OR PRIMARY ALCOHOLS
PROCEDE ET DISPOSITIF PERMETTANT DE PRODUIRE DES ACIDES MONOCARBOXYLIQUES A PARTIR D'HYDRATES DE CARBONE, DE DERIVES D'HYDRATE DE CARBONE OU D'ALCOOLS PRIMAIRES

(30) Priorität: 10.03.1993 DE 4307388
(43) Veröffentlichungstag der Anmeldung: 10.05.1995
(73) Patentinhaber: SÜDZUCKER AKTIENGESELLSCHAFT MANNHEIM/OCHSENFURT, D-68165 Mannheim (DE)
(72) Erfinder: KUNZ, Markwart, D-38104 Braunschweig (DE); PUKE, Hanjo, D-38110 Braunschweig (DE); RECKER, Carla, D-38102 Braunschweig (DE); SCHEIWE, Linda, D-38108 Braunschweig (DE); KOWALCZYK, Jörg, D-67269 Grünstadt (DE)
(74) Vertreter: Einsel, Martin
(86) Internationale Anmeldenummer: EP9400695
(87) Internationale Veröffentlichungsnummer: WO9420448

(56) Entgegenhaltungen:
- EP-A- 0 040 709
- EP-A- 0 186 836
- EP-A- 0 206 054
- EP-A- 0 326 673
- PATENT ABSTRACTS OF JAPAN vol. 9, no. 184 (C-294)(1907) 30. Juli 1985 & JP,A,60 054 338 (MITSUI TOATSU KAGAKU K.K.) 28. März 1985

## Beschreibung

Die Erfindung betrifft ein Verfahren und eine Vorrichtung zur Herstellung von Monocarbonsäuren von Kohlenhydraten, Kohlenhydratderivaten oder primären Alkoholen.

Mit verschiedenen biochemischen Verfahren gelingt es, Kohlenhydrate selektiv zu oxidieren. Diese Herstellungsmethoden weisen jedoch erhebliche Nachteile auf. Zum einen ist die Aufzucht von Mikroorganismen bzw. Herstellung von Biokatalysatoren mit erheblichen Schwierigkeiten verbunden. Bei den Produktionsverfahren handelt es sich meistens um Fermentation (z.B. bei der Herstellung von Gluconsäuren) so daß hier der Einsatz von Nährsalzen in der Fermentationslösung zu erheblichen Salzfrachten führt. Ein weiterer Nachteil ist die häufig notwendige sterile Fahrweise bei diesen Prozessen, so daß mit erheblichen Anlagekosten gerechnet werden muß.

Besondere Bedeutung hat die heterogen katalysierte Oxidation mit Edelmetallen der 8. Nebengruppe auf entsprechenden Trägermaterialien erlangt. Die Oxidation von Glucose zu Gluconsäure mit Luftsauerstoff kann auf diese Weise chemisch z.B. an Pt/C-Katalysatoren durchgeführt werden. Nachteilig bei dieser Verfahrensweise ist jedoch die stark abnehmende Selektivität der Reaktion sowie die schnelle Deaktivierung des Katalysators, vgl. Ullmanns Enzyklopädie der Technischen Chemie, 4. neu bearbeitete Auflage Band 24, Seite 785 Verlag Chemie 1983.

Ähnliche Probleme werden bei der Oxidation von Saccharose beobachtet. Bereits Heyns und Paulsen untersuchten diese Reaktion an Platinkatalysatoren (K.Heyns, H Paulsen, Adv. Carbohydr. Chem. 17, 169 (1962); K.Heyns, W.D.Soldat, P.Köll, Chem. Ber., 3619 (1975)). Als resultierende Produkte erhielten sie Mischungen von oxidierten Verbindungen, zu denen hinsichtlich chemischer Strukturen und Zusammensetzungen aufgrund der Komplexität keine näheren Angaben gemacht wurden.

Bei einem aus der EP 0 040 709 B1 bekannten Verfahren zur Herstellung von Diacetonketogulonsäure wird Diacetonsorbose teiloxidiert und mittels Elektrodialyse aufgetrennt; hierbei handelt es sich um ein diskontinuierliches Verfahren und durch Einführung von Schutzgruppen besitzt das Ausgangsderivat nur eine oxidierbare Gruppe.

Aus der DE 38 03 465 A1, der DE 39 16 206 A1 und der US-PS 4 985 553 sind verschiedene Batch-Prozesse bekannt, die quantitativ nicht befriedigende Umsatzzahlen bzw. Produktmischungen mit nicht zufriedenstellenden Verunreinigungen mit Ausgangsverbindungen liefern. Teilweise wird in den Entgegenhaltungen bereits vorgeschlagen, mit aufwendigen Reinigungsverfahren eine Produktisolierung vorzunehmen.

Ein in der EP 0 218 150 B1 beschriebenes Verfahren zur katalytischen Oxidation von Saccharose weist ausdrücklich darauf hin, daß bei diskontinuierlicher Prozeßführung im hohen Maße mehrfach oxidierte Produkte erhalten werden.

Versuche, Saccharose selektiv an nur einer primären OH-Gruppe zu oxidieren, führten bisher mit den herkömmlichen Technologien nicht zum Ziel.

Es treten neben den 3 möglichen, durch Oxidation der primären OH-Gruppen erhältlichen, Monocarbonsäuren ebenfalls die Di- und Tri- Carbonsäuren als Verbindungen im Produkt-Gemisch auf. Außerdem werden bei den beschriebenen Varianten eine Reihe von nicht näher spezifizierten Spaltprodukten gebildet, die zu erheblichen Ausbeuteverlusten führen und die Selektivität der Reaktion hinsichtlich der Bildung von Monocarboxysäuren erheblich mindern. (Les A. Edye, George V. Meehan, Geoffrey N. Richards, Platinum catalysed oxidation of sucrose, J. Carbohydrate Chemistry, 10 (1), 11-23 (1991)).

Das gleiche wurde auch bei reduzierenden Sacchariden beobachtet, wie z.B. Untersuchungen an Palatinose belegen (Dissertation H. Puke, TU Braunschweig).

Aufgabe der Erfindung ist es demgegenüber, eine Oxidation von Kohlenhydraten, Kohlenhydratderivaten und primären Alkoholen mit einer besseren Selektivität bezüglich der monooxidierten Produkte vorzuschlagen.

Diese Aufgabe wird dadurch gelöst, daß man kontinuierlich Kohlenhydrate, Kohlenhydratderivate oder primäre Alkohole in wäßriger Lösung und Konzentration zwischen 0,1 und 60% mit Sauerstoff oder sauerstoffhaltigen Gasen an Edelmetall- oder Mischmetallkatalysatoren cxidiert, den Volumenstrom der so gebildeten Produkte einer Elektrodialyse zuführt und die Monocarbonsäure entfernt und gewinnt.

Dieses Verfahren ist zur Herstellung von monooxidierten Kohlenhydraten bzw. Kohlenhydratderivaten und primären Alkoholen in ausgezeichneter Weise geeignet. Die geschickte, kontinuierliche Reaktionsführung sowie die gleichzeitige Abtrennung der Oxidationsprodukte mittels Elektrodialyse führt tatsächlich zum Erhalt von praktisch ausschließlich Monocarboxy-Verbindungen von Kohlenhydraten bzw. Kohlenhydratderivaten oder primären Alkoholen. Dabei ist gleichzeitig eine hohe Raum-Zeitausbeute möglich.

Die spezifische Funktionalisierung von Sacchariden bzw. Saccharidderivaten, die unter die Ausgangsstoffe fallen, ist für die Synthese von hydrophilen Bausteinen für den Polymer- und Tensidsektor auf Kohlenhydratbasis von hohem industriellen Interesse. Aufgrund ihrer ökologisch positiven Eigenschaften weisen diese Rohstoffe gegenüber synthetischen Produkten erhebliche Vorteile auf.

Als besonders effektiv hat es sich erwiesen, wenn die nach der Elektrodialysestufe und dem Entzug der Monocarbonsäuren verbleibenden Stoffe wieder der Oxidationsstufe zugeführt werden. Es entsteht so ein kontinuierlicher Kreislauf und eine besonders effektive Aufarbeitung der Ausgangsstoffe.

Günstig ist es, wenn der Stoffstrom vor Betreten des Katalysatorbettes mit Luft blasenfrei angereichert wird, so daß genügend Sauerstoff für die Oxidationsreaktion verfügbar ist.

Die hier vorliegende Erfindung beschreibt ein kontinuierliches Verfahren, bei dem Kohlenhydrate bzw. Kohlenhydratderivate durch Kombination zweier Verfahrensschritte selektiv zu Monocarboxyderivaten umgesetzt werden können. Die erste Verfahrensstufe besteht aus einer kontinuierlich betriebenen Oxidation an Edelmetall- oder Mischmetallkatalysatoren. Letztere eignen sich ebenfalls, jedoch im Hinblick auf die Recyclingfähigkeit sind Edelmetall-katalysatoren mit einem katalytisch aktiven Element zu bevorzugen.

Anschließend werden die bei der Oxidation gebildeten Monocarbonsäuren, ebenfalls kontinuierlich durch den zweiten Verfahrensschritt, eine Elektrodialysastufe, aus dem Reaktionsgemisch entfernt. Diese bisher noch nicht beschriebene Kombination von kontinuierlich betriebener Oxidation mit anschließender kontinuierlicher Entfernung der gebildeten Oxidationsprodukte eignet sich in besonderer Weise zur Herstellung der Monocarbonsäuren von Kohlenhydraten bzw. deren Derivaten. Es werden hierbei höhere Umsätze als bei den bisher beschriebenen Verfahren erzielt und die Selektivität, bezogen auf die Bildung von Monocarbonsäuren liegt überraschenderweise über 95%.

Die kontinuierlich betriebene Oxidation wird verfahrenstechnisch folgendermaßen gelöst:
a) Das Reaktorsystem besteht aus einer Begasungsstufe (Rührkessel) sowie einem Rohrreaktor, in dem der Festbettkatalysator angeordnet ist. Die Begasungsstufe kann insbesondere ein Rührkessel (Rührreaktor) sein. Im Rührreaktor erfolgt die Sauerstoffanreicherung, entweder durch Einleiten von Luft, bzw. Gasgemischen mit höheren Sauerstoffpartialdrücken oder reinem Sauerstoff als fein verteilte Blasen bzw. blasenfrei über spezielle Gaseinleitungsschläuche, unter Druck oder drucklos. Dieser Rührreaktor ist mit einem parallel angeordneten Rohrreaktor verbunden (Up- und Downflow möglich), in dem die eigentliche Oxidation am Katalysatorkontakt stattfindet.
b) Die Oxidation kann ebenfalls mit suspendierten Katalysatoren (Slurryverfahren) in einem Rührkessel durchgeführt werden, wobei die Anbindung an die Elektrodialyseeinheit über eine Separationsstufe verläuft. Im einfachsten Fall können hierfür Dekanterzentrifugen bzw. Cross-Flow Module eingesetzt werden. Die technologische Lösung dieses Problemes gelingt auch mit entsprechendem Rückhaltesystemen wie Filtern mit Rückspüleinrichtung, Separatoren usw.

Als Katalysatoren eignen sich Edelmetallkontakte oder Mischmetallkatalysatoren, die z.B. als Extrudate (C, Oxide), Fasern, Tabletten oder Pulver eingesetzt werden können. Bei Verwendung von Edelmetallkatalysatoren sollte der Metallanteil zwischen 0,1 und 10 % liegen. Besonders gute Ergebnisse konnten mit Pt-Katalysatoren erzielt werden, die einen Platingehalt von nur 1% aufwiesen und als Pulver vorlagen, wobei jedoch der Feinanteil duch Klassierung entfernt wurde. Der aus dem Rohrreaktor kommende Teilstrom wird anschließend der Elektrodialyse auf der Diluatseite zugeführt, so daß die oxidierten Produkte durch Anlegen einer Spannung in das Konzentrat wandern und anschließend aus dem Reaktionssystem ausgeschleust werden. Um den Gleichgewichtszustand des kontinuierlich betriebenen Reaktionssystemes aufrecht zu erhalten, wird regelungstechnisch die Menge, die aus dem Konzentrat ausgeschleust wird, durch Zugabe von Eduktlösung in den Rührreaktor nachdosiert.

Mischmetallkatalysatoren sind beispielsweise solche, wie sie von der Degussa AG angeboten und in einem Aufsatz von K.Deller und B.Despeyroux in: "Catalysis of organic reactions" (1992) beschrieben werden.

Eine besonders geeignete Vorrichtung zur Durchführung des erfindungsgemäßen Verfahrens zeichnet sich dadurch aus, daß sie in Reihe geschaltet eine Begasungsstufe, eine Oxidationsstufe und eine Elektrodialysestufe aufweist.

Zur besonders genauen Abstimmung der jeweils verarbeitbaren Stoffmengen ist es besonders bevorzugt, wenn parallel zur Elektrodialysestufe eine Zweigleitung vorgesehen ist.

Mit dieser zusätzlichen parallelen Leitung entsteht eine Art Kurzschlußweg parallel zur Elektrodialysestufe. Zusammen mit entsprechenden Durchflußsteuereinheiten bzw. Pumpen ist es möglich, aus dem Katalysator bzw. der vorgeschalteten Begasungsstufe zugeführte Stoffmengen der Elektrodialysestufe nur insoweit zuzuführen, als sie dort verarbeitet werden können und nicht verarbeitbare Mengen durch den Kurzschlußweg direkt wiederum in die Begasungsstufe zurückzuführen. In jeder der Stufen wird damit genau die optimale Stoffmenge bearbeitet.

Die Namen der drei möglichen Saccharose-Monocarbonsäuren werden in dieser Anmeldung nur als Kurzbezeichnungen geführt. Die vollständigen Namen nebst denen zweier weiterer im folgenden noch erwähnter Produkte lauten:
C₆-Saccharosemonocarbonsäure: 1-0-(β-D-Fructofuranuronyl) -α-D-glucopyranosid
C₁-Saccharosemonocarbonsäure: 2-Keto-2-0-(α-D-glucopyranosyl)-β-D-glucofuranonsäure
C₆,-Saccharosemonocarbonsäure: 1-0-(β-D-Fructofuranosyl)-α-D-glucopyranuronid
C₁-oxidiertes GMF: 5-(α-D-Glucopyranosyl-oxymethyl)-furan-2-carbonsäure
C₆,-oxidiertes GMF: 5-(α-D-Glucopyranuronyl-oxymethyl)-furfural

Im folgenden werden anhand von Zeichnungen eine Vorrichtung zur Durchführung des Verfahrens und dabei zugleich besonders bevorzugte Parameter näher erläutert.

Es zeigen:
Fig. 1 eine bevorzugte Ausführungsform der Erfindung;
Fig. 2 einen Verfahrensablauf in einem ersten Beispiel;
Fig. 3 einen Verfahrensablauf in einem zweiten Beispiel;
Fig. 4 eine alternative Ausführungsform zu Fig. 1.

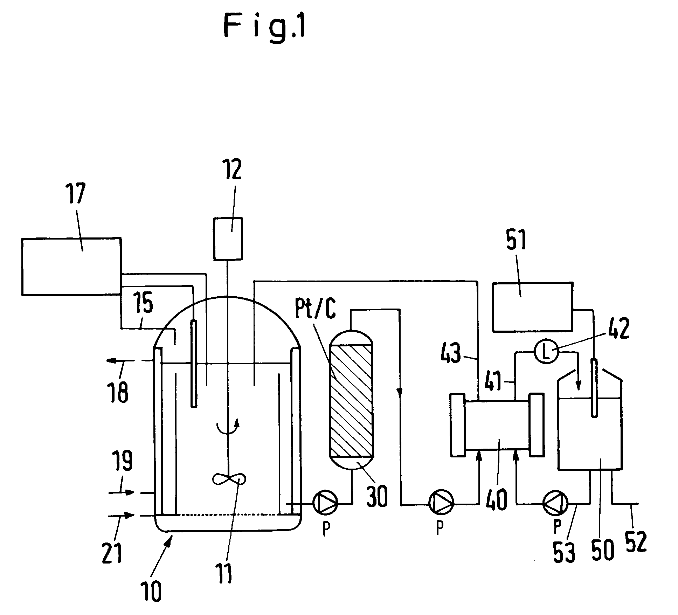

Die schematische Darstellung in Fig. 1 zeigt ein Rührgefäß bzw. einen Behälter 10 mit einem Rührer 11 und einem Motor 12 für den Rührer 11. In den Behälter 10 wird bei 15 das Edukt hineingegeben, bei 17 ist schematisch eine pH-Regelung vorgesehen, bei 18 werden Daten an einen Thermostaten aus- und bei 19 von diesem eingegeben. Außerdem wird bei 21 Luft (N₂/O₂) dem Behälter 10 zugeführt.

Nach der Durchmischung im Rührgefäß wird über eine der mit P bezeichneten Pumpen das mit Sauerstoff angereicherte und durchmischte, pH-geregelte Edukt, etwa ein Kohlenhydrat, der Oxidationsstufe 30 zugeführt. Diese enthält einen Edelmetall- oder Mischmetallkatalysator, in diesem Fall einen Pt/C-Trägerkontakt. In der Oxidationsstufe 30 findet eine kontinuierliche Oxidation des Ausgangstoffes statt; er wird danach über die nächste der mit P bezeichneten Pumpen zu einer Elektrodialysestufe 40 geführt. Diese, ein ED-Stack bzw. eine Elektrodialysezelle, ist ebenfalls nur schematisch dargestellt. In ihr wird kontinuierlich aus der teilweise oxidierten Mischung die Monocarbonsäure abgeführt und zwar über den mit 41 bezeichneten Weg, in dem sich auch eine Leitfähigkeitsmeßzelle 42 befindet, als "L" zusätzlich gekennzeichnet.

Die nichtmonooxidierten Kohlenhydrate etc. werden dagegen über den Weg 43 wieder in den Behälter 10 zur weiteren Verarbeitung zusätzlich zum Edukt 15 zurückgeführt, dem sie chemisch ohnehin entsprechen.

Die Monocarbonsäure werden nach Durchlaufen der Leitfähigkeitsmeßzelle 42 in einen Behälter 50 geführt und dort konzentriert. In dem Behälter 50 findet ständig eine bei 51 angedeutete pH-Messung statt. Dabei wird hier bei 52 das Produkt abgeführt, bei 53 wird über eine Pumpe P wiederum nicht ausgeschleustes Produkt in die Elektrodialysestufe zurückgeführt.

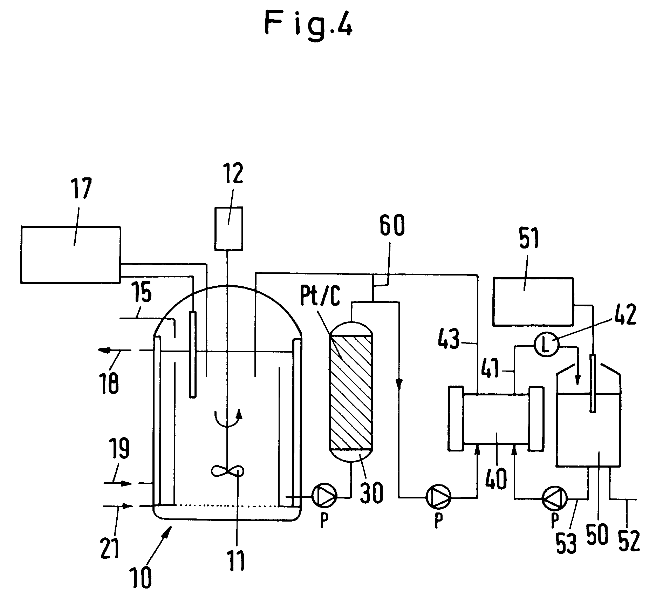

In Fig. 4 ist eine in fast allen Einzelheiten mit Fig. 1 übereinstimmende Darstellung angegeben. Zusätzlich ist noch eine Leitung 60, ein Kurzschlußweg zwischen dem Ausgang der Oxidationsstufe 30 und der Rückleitung 43 von der Elektrodialysestufe in den Behälter 10 vorgesehen.

Diese Leitung 60 ist hier rein schematisch; sie kann zusätzliche Behälter, Meßstufen und -einrichtungen, Pumpen und Durchflußsteuerungselemente enthalten.

Die bereits erwähnten Pumpen P, die auch in der Ausführungsform nach Fig. 1 enthalten sind, sind allein oder mit diesen genannten Elementen in der Lage, von der Oxidationsstufe 30 in die Elektrodialysestufe 40 nur solche Stoffmengen zu überführen, die dort auch optimal verarbeitet werden können. Überschußmengen können über den Kurzschlußweg, die Leitung 60, wieder in die Begasungsstufe bzw. den Behälter 10 zurückgeführt werden, zusammen mit den nicht monooxidierten Kohlenhydraten aus der Elektrodialysestufe 40 über den Weg 43.

Als Beispiel sei Saccharose als nichtreduzierendes Disaccharid diskutiert, die mit dem beschriebenen Verfahren in der dargestellten Vorrichtung selektiv zu ihren Monocarbonsäuren umgesetzt werden kann.

Obwohl pro Molekül drei primäre Hydroxygruppen zur Reaktion befähigt sind, erfolgte die Oxidation an nur jeweils einer dieser Gruppen, so daß ausschließlich monooxidierte Saccharosederivate erhalten werden. Die Selektivität zu diesen Produkten liegt bei mind. 95% und die mögliche Bildung der Di- bzw. Tricarbonsäure wird bei Einsatz dieses kontinuierlichen Verfahrens nicht beobachtet.

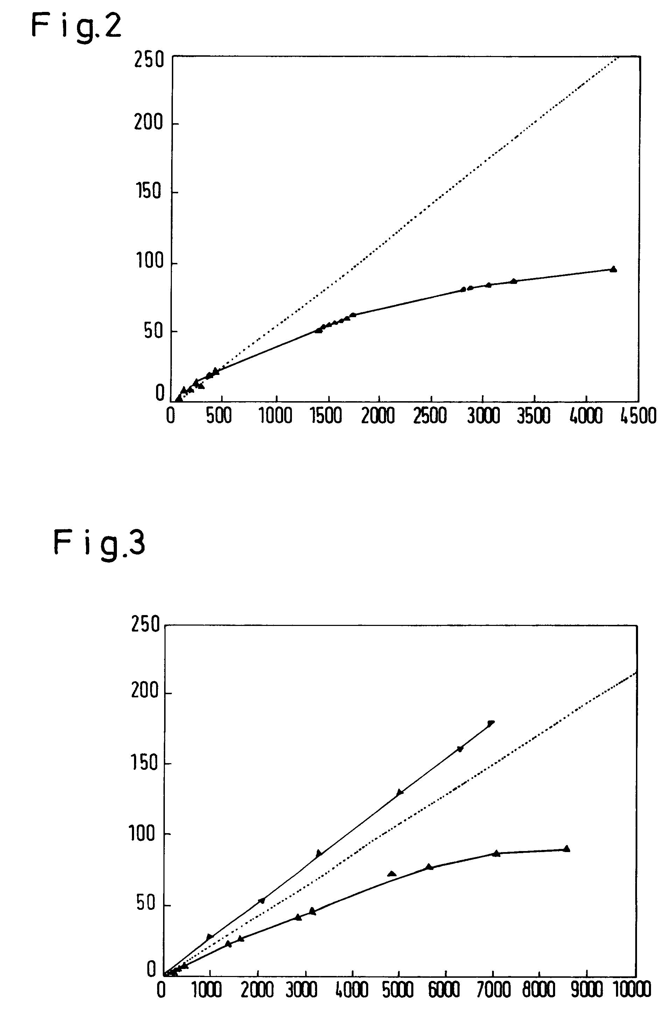

Neben der hohen Selektivität kann im Vergleich zum diskontinuierlichen Verfahren bei dieser kontinuierlichen Betriebsweise ein erheblicher Anstieg der Reaktionsgeschwindigkeit beobachtet werden. Die Vorteile des erfindungsgemäßen Verfahrens bezüglich der Reaktivität sind für Glucose und Saccharose, verglichen mit der diskontinuierlichen Betriebsweise, in Fig. 2 und 3 dokumentiert.

In den Figuren 2 und 3 ist jeweils horizontal die Zeit in Minuten aufgetragen; vertikal der Umsatz in Prozent. Graphisch ist jeweils mit dem Symbol Δ ein diskontinuierlicher Versuch eingetragen, in Fig. 2 für Glycoseumsatz, in Fig. 3 für Saccharoseumsatz. Strichpunktiert ist ein kontinuierlicher Versuch, wiederum in Fig. 2 für Glucoseumsatz und in Fig. 3 für Saccharoseumsatz eingetragen; in Fig. 3 zusätzlich noch ein kontinuierlicher Versuch mit reinem Sauerstoff (O₂).

Eine weitere Steigerung der Reaktionsgeschwindigkeit hinsichtlich der Bildung von Monocarbonsäuren kann durch Erhöhen des Sauerstoffpartialdruckes in der Lösung z.B. durch Einleiten von reinem Sauerstoff (anstatt Luft oder Sauerstoff/Stickstoffmischungen) erzielt werden.

Ein weiterer technischer Vorteil dieser erfindungsgemäßen Betriebsweise ist, daß eine Deaktivierung des Katalysators, wie sie im allgemeinen beim diskontinuierlichen Betrieb auftritt (Vergleich: K.Heyns, H.Paulsen (siehe vorn); H.Puke, Dissertation TU Braunschweig) nicht beobachtet wird. Selbst beim Einleiten des reinen Sauerstoffes findet überraschenderweise keine Desaktivierung des Katalysators statt. Dieser Vorteil ist in der einschlägigen Literatur bisher noch nicht beschrieben und erweist sich auch in technologischer Hinsicht als großer Fortschritt bei der gezielten Derivatisierung von Kohlenhydraten.

Das Verfahren kann problemlos auf reduzierende Zucker übertragen werden, wie z.B. Palatinose und Glucose.

Die Selektivität zu bestimmten Monooxidationsprodukten kann durch Wahl geeigneter Katalysatoren, entweder durch Verwendung bestimmter Träger oder Mischmetallkontakte, bzw. durch den pH-Wert gesteuert werden.

Bei der Palatinose ist es außerdem möglich, durch Verwendung von speziellen Edelmetall-Katalysatoren (z.B. Pt/Al₂O₃ (1% Pt) Fa. Aldrich), bei denen als Träger Al₂O₃ eingesetzt wird, weitestgehend die primäre OH-Gruppe in 6'-Position zu oxidieren.

In diesem Fall zeigt sich, daß trotz der Möglichkeit zur Bildung der Dicarbonsäure, ausschließlich nur die Monocarbonsäure entsteht. Weiterhin ist zu beobachten, daß durch die Wahl des Katalysators bei diesem Verfahren die Selektivität so beeinflußt werden kann, daß die Oxidation überraschenderweise im wesentlichen nur zu einem Oxidationsprodukt führt.

Auch Versuche mit anderen Katalysatoren zeigten, daß man nicht nur die Selektivität zu den Monosäuren sondern auch die Selektivität zu einem gewünschten Produkt bei diesem Verfahren steuern kann. Das Verfahren eignet sich nicht nur zur Oxidation von Sacchariden, sondern es ist auch möglich, Zuckeralkohole (z.B. Isomalt) in die korrespondierenden Monosäuren zu überführen.

Weiterhin ist es möglich Kohlenhydratderivate, wie z.B. Glucopyranosylmethylfurfural zu oxidieren, wobei zum einen die 6'Position sowie zum anderen die Aldehydfunktion in die entsprechende Monocarbonsäure überführt wird.

Auch bei diesem Edukt werden keine zweifachoxidierten Produkte erhalten. Trotz des Vorhandenseins einer leichter oxidierbaren Aldehydfunktion werden ebenfalls Produkte isoliert, die ausschließlich in C_{6'}-Position eine Carboxylfunktion tragen. Die Aldehydunktion bleibt bei diesen Verbindungen erhalten.

Ebenfalls können Alkylglycoside bzw. Mischungen, wie z.B. Alkylpolyglycoside mit diesem Verfahren oxidiert werden.

Die Beispiele belegen, daß sich das hier beschriebene Verfahren zur Oxidation von Aldehyd- und primären Hydroxyfunktionen eignet, um monooxidierte Produkte zu erhalten. Die eingesetzten Edukte, die hier hauptsächlich aus dem Bereich der Kohlenhydrate stammen, müssen in Wasser bzw. in Mischungen von Wasser und organischen Lösungsmitteln (z.B. Wasser- Isopropanolmischungen) löslich sein und sich bei den verwendeten Versuchsbedingungen nicht verflüchtigen. Auch bei Edukten aus dem "Nichtkohlenhydrat-Sektor" eignet sich das Verfahren zur Herstellung von monooxidierten Produkten (z.B. Propanol zu Propansäure), solange sie (auch teilweise) in den beschriebenen Medien löslich sind.

Die Oxidation erfolgt bei Temperaturen zwischen 0-80°C, vorzugsweise jedoch zwischen 20 und 60°C. Die Eduktkonzentrationen können zwischen 0,1-60% variieren, werden jedoch vorzugsweise im Bereich von 3-20% gehalten. Die pH-Werte können bei der Oxidation durch Zugabe von Na₂CO₃, NaHCO₃ bzw. NaOH oder andere "Alkalisierungsmittel" im Bereich von 1-13 eingestellt werden.

Zur Isolierung der Monocarbonsäuren können in der Elektrodialyse Ionenaustauschermembranen eingesetzt werden. Hierbei können jedoch nur bei niedrigen pH-Werten die freien Säuren gewonnen werden. Bei neutraler Betriebsweise werden hierbei jedoch meistens die Na-Salze der Monocarbonsäuren isoliert.

Führt man die ED mit bipolaren Membranen durch, so kann man das Neutralisierungsmittel wieder zurückgewinnen und außerdem die freien Monocarbonsäuren der korrespondierenden Edukte erhalten. Für bipolare Membranen müssen zwar höhere Investitionskosten kalkuliert werden, jedoch muß die Wirtschaftlichkeit bezüglich der Weiterverarbeitung in Hinsicht auf nachfolgende Operationen von Fall zu Fall überprüft werden.

Vergleichsversuche mit einer nichtkontinuierlichen Betriebsweise (Batch) belegen eindeutig die Vorteile des hier beschriebenen Verfahrens. Bei den Batch-Versuchen sind die Reaktionsgeschwindigkeiten bedeutend kleiner. Die Selektivität bei den Vergleichsversuchen zu den Monocarboxyverbindungen nimmt deutlich ab und es treten in erheblichem Maße Nebenprodukte auf, die nicht näher identifiziert wurden.

Bei der kontinuierlichen Arbeitsweise wurde bei dem hier beschriebenen Verfahren selbst nach einer Zeit von 2 Monaten keine Deaktivierung des Katalysators beobachtet. Bei der Batch-Arbeitsweise kommt es schon nach kurzer Zeit zur Inaktivierung des Katalysators, wie auch schon in der Literatur beschrieben ist.

Zur Durchführung der Oxidation wird die in Fig. 1 beschriebene Umlaufapparatur eingesetzt. Die Begasung erfolgt in einem rührbaren zylindrischen Doppelwandrührreaktor (500 ml) über einem Frittenboden. Als Beruhigungszone ist ein Glaseinsatz eingebaut, von der ein Teilstrom mit einer Kreiselpumpe durch das Katalysatorbett, das sich in einer mit 2 Fritten abgeschlossenen Glassäule befindet, gefördert wird. Nach passieren des Festbettes gelangt dieser Teilstrom zur Elektrodialyseeinheit und wird nach Abtrennung der Oxidationsprodukte anschließend wieder in den Rührreaktor zurückgeführt. Das Produkt wird über den Konzentratkreislauf der Elektrodialyse ausgeschleust und die äquivalente Menge Eduktlösung wird mit Hilfe einer Schlauchpumpe in den Rührreaktor nachdosiert. Das Lösungsdefizit beim Konzentratkreislauf wird duch destilliertes Wasser ersetzt.

### Beispiel 1

Kontinuierliche Oxidation von Palatinose bei 35°C.

In die beschriebene Apparatur werden 20 g Platin/Aktivkohlekatalysator (5% Pt/C; Korngröße 40-100 µm, Fa. Degussa) und 1000 ml einer 0,1 molaren Palatinoselösung in die Umlaufapparatur und den Diluatkreislauf der Elektrodialyse eingefüllt. Für den Konzentrationskreislauf wird destilliertes Wasser und als Elektrodenspülung 1 M Na₂SO₄ eingesetzt.

Die Temperatur wird durch einen Umlaufthermostaten bei 35°C gehalten, die Gaszufuhr (N₂/O₂, 4:1) kann über Druckminderer und Nadelventile eingestellt und der Gasvolumenstrom mit einem Rotameter gemessen werden, so daß die Begasungsrate von 100 cm³/min. O₂ und 400 cm³/min. N₂ eingehalten wird. Der pH-Wert wird durch Titrieren der entstandenen Säure mit 1 M NaHCO₃ bei 6,5 konstant gehalten. Die Elektrodialyse (Bel II, Fa. Berghof GmbH Labortechnik) ist mit 6 AMV/CMX Membranpaaren (Eff. Membranfläche = 360 cm) ausgestattet und wird bei einer Spannung von 5-6 V betrieben. Nach Erreichen des Gleichgewichtszustandes wird der Reaktionsverlauf durch HPLC-Messungen verfolgt. Das Produktspektrum der erhaltenen Substanzen hat im Konzentrat folgende Zusammensetzung:
6-O-(α-D-glucopyranuronyl)-D-fructofuranose (C₆,-Säure): 50%
2-Keto-6-O-(α-D-glucopyranosyl)-D-arabino-hexonsäure (C₁- Säure): 42,5%
5-O-(α-D-glucopyranosyl)-D-arabonsäure (GPA-Säure): 3,5%
Selektivität zu den Monocarbonsäuren: 96%

Die Substanzen konnten im präparativen Maßstab getrennt und mittels NMR- und Massenspektrometrie charakterisiert werden.

### Beispiel 2

### Oxidation der Palatinose bei 42,5°C

In Analogie zu Beispiel 1 wird Palatinose bei einer Reaktionstemperatur von 42,5°C oxidiert. Das erhaltene Produkt-gemisch im Konzentrat hat folgende Zusammensetzung:

| | |
|---|---|
| C_{6'}-Säure | 50,0% |
| C₁-Säure: | 42,5% |
| GPA-Säure: | 4,0% |
| Selektivität zu Monosäuren: | 96,5% |

### Batch-Vergleichsversuch 1

### Oxidation der Palatinose

Die Durchführung der Batch-Oxidation erfolgt ebenfalls in dem Umlaufreaktorsystem, jedoch ohne Elektrodialyse und Eduktzudosierung.

36 g Palatinose werden in 1000 ml dest. Wasser gelöst und bei 35°C oxidiert. Als Katalysator wird ein Platin/Aktivkohle Typ (5% Pt, Fa. Degussa, 40-100 µm) verwendet.

Bei einem Palatinose-Umsatz von 80% wird der Versuch nach 4 Tagen abgebrochen und nichtoxidierte Produkte mittels Chromatographie abgetrennt.

Das erhaltene Produkt hat folgende Zusammensetzung:

| | |
|---|---|
| C_{6'}-Säure: | 50,6% |
| C₁-Säure: | 23,5% |
| Di-Säure: | 8,1% |

mono sowie einen erheblichen Anteil nichtidentifizierter Produkte. Die Selektivität hinsichtlich der Bildung von Monocarboxysäuren liegt in diesem Fall nur bei 74,1%.

### Beispiel 3

### Oxidation von Glucose

Die Oxidation der Glucose erfolgt in der in Beispiel 1 beschriebenen Apparatur, bei einem pH-Wert von 6,5 (Zugabe von NaHCO₃) und bei einer Temperatur von 35°C. Die kontinuierlich erhaltene Produktlösung hat hier die folgende Zusammensetzung:

| | |
|---|---|
| Gluconsäure-Na-Salz: | 92% |
| Glucuronsäure-Na-Salz: | 7% |

Die Selektivität bezogen auf die Monosäuren liegt bei 99%.

### Batch-Vergleichsversuch 2

Die Umsetzung der Glucose bei 35°C, einem pH-Wert von 6,5 und Verwendung des Platin/Aktivkohlekatalysators (5% Pt, Fa. Degussa, 40-100 µm) führte nach 3 Tagen zu einem Umsatz von ca. 80%.

### Die oxidierten Hauptprodukte sind

| | |
|---|---|
| Gluconsäure-Na-Salz: | 60% |
| Glucuronsäure-Na-Salz: | 15% |
| Glucarsäure-Na-Salz: | 10% |

sowie 15% nicht näher identifizierte Produkte.

Die Selektivität bezüglich der Monosäuren liegt hier bei 75%.

Die graphische Darstellung in Fig. 2 zeigt den Reaktionsverlauf der kontinuierlichen und absatzweisen Oxidation der Glucose. Die Reaktivität der kontinuierlichen Fahrweise ist bedeutend höher und Deaktivierung des Katalysators ist nicht zu beobachten.

### Beispiel 4

### Oxidation der Glucose bei pH 3

Die Oxidation erfolgte wie im Beispiel 3 beschrieben, jedoch erfolgt keine feste pH-Einstellung durch Zugabe von NaHCO₃. Nach der Anlaufphase liegt der pH-Wert durch die sich bildenden Säuren bei 3.

Unter diesen Reaktionsbedingungen bildet sich aus Gluconsäure das Gluconsäure δ -Lacton, das jedoch durch Anheben des pH-Wertes wieder in Gluconsäure überführbar ist.

### Produktzusammensetzung:

| | |
|---|---|
| Gluconsäure: | 60% |
| Gluconsäure δ-Lacton: | 20% |
| Glucuronsäure: | 15% |

### Beispiel 4a

Mischmetallkatalysatoren sind Edelmetallkatalysatoren, die zwei oder mehrere katalytisch wirksame Metalle mit Promotoren wie z.B. Wismut enthalten. Ein Problem bei diesen Katalysatoren im Batch-Versuch ist, daß sie bei längeren Reaktionszeiten nicht mehr so selektiv sind. Hierbei handelt es sich jedoch um Folgereaktionen mit den Monocarbonsäuren, so daß erhebliche Mengen an Nebenprodukten anfallen. Durch die Verwendung des erfindungsgemäßen Verfahrens kann dieser Nachteil beseitigt werden. Die gebildeten Produkte werden unverzüglich nach der Bildung mittels Elektrodialyse entfernt und kommen somit nicht mehr mit dem Kontakt in Berührung.

In einem praktischen Beispielversuch mit einem Pt/Pd/Bi-Mischmetallkatalysator (Degussa) konnte dieses anhand der Oxidation der Glucose zu Gluconsäure bzw. Glucuronsäure belegt werden.

Die Oxidation erfolgte dabei wie in Beispiel 3 beschrieben, abgesehen von dem Einsatz des Pt/Pd/Bi-Mischmetallkatalysators.

### Produktzusammensetzung:

| | |
|---|---|
| Gluconsäure-Na-Salz: | 94% |
| Glucuronsäure-Na-Salz: | 4% |

Die Selektivität bezüglich der Monosäuren liegt bei 98%.

### Beispiel 5

### Oxidation der Saccharose

Nach der in Beispiel 1 beschriebenen Verfahrensweise läßt sich auch Saccharose in die korrespondierenden Monocarbonsäuren überführen.

Die Oxidation erfolgt bei einem pH-Wert von 6,5 und einer Temperatur von 35°C, wobei als Produkt eine Mischung aus drei Monocarbonsäuren erhalten wird.

| | |
|---|---|
| C_{6'}-Saccharosemonocarbonsäure: | 46,5 |
| C₆-Saccharosemonocarbonsäure: | 43,7 |
| C₁-Saccharosemonocarbonsäure: | 4,9 |

Die Selektivität liegt bei über 95%.

### Batch-Vergleichsversuch 3

### Oxidation der Saccharose

Die diskontinuierliche Oxidation bei 35°C, einem pH-Wert von 6,5 führte nach 6 Tagen bei einem Umsatz von 90% zu folgendem Produktspektrum.

| | |
|---|---|
| C_{6'}-Saccharosecarbonsäure: | 40,0% |
| C₆-Saccharosecarbonsäure: | 31,0% davon ca. 10% Disäure |
| C₁-Saccharosecarbonsäure: | 8,8% |

Die Selektivität zu den Monocarboxyverbindungen liegt bei ca. 70%.

Die graphische Darstellung in Fig. 3 zeigt die Vorteile der kontinuierlichen Arbeitsweise, bezüglich der Reaktionsgeschwindigkeit.

### Beispiel 6

### Oxidation von Glucopyranosylmethylfurfural (GMF)

Die kontinuierliche Oxidation von GMF erfolgt nach der gleichen Verfahrensweise wie unter Beispiel 1 beschrieben.

Bei einer Temperatur von 35°C und einem pH-Wert von 7 erhält man

| | |
|---|---|
| C_{6'}-oxidiertes GMF: | 33% |
| C₁-oxidiertes GMF: | 66% |

Die Selektivität zu den monooxidierten Produkten liegt bei 99%.

### Beispiel 7

Die Oxidation der Saccharose erfolgt in der in Beispiel 1 beschriebenen Apparatur, bei einem pH-Wert von 6,5 und bei einer Temperatur von 35°C. Anstatt des Sauerstoff-Stickstoffgemisches wird reiner Sauerstoff eingeleitet, wodurch die Bildung an oxidierten Produkten entsprechend beschleunigt wird (Fig. 3).

Die oxidierten Hauptprodukte sind:

| | |
|---|---|
| C_{6'}-Saccharosecarbonsäure: | 43,0% |
| C₆-Saccharosecarbonsäure: | 43,0% |
| C₁-Saccharosecarbonsäure: | 9,5% |

Die Selektivität liegt bei über 95%.

Zusammengefaßt betrifft die Erfindung ein Verfahren und eine Vorrichtung zur Herstellung von monooxidierten Produkten aus Kohlenhydraten, Kohlenhydratderivaten und primären Alkoholen. Die Ausgangsstoffe werden einer kontinuierlich betriebenen Oxidationsstufe mit Edelmetall- oder Mischmetallkatalysatoren zugeführt. Der Strom mit den an den Katalysatoren gebildeten Monocarbonsäuren wird einer Elektrodialysestufe zugeführt, wobei kontinuierlich die Monocarbonsäuren entfernt und gewonnen werden.

## Patentansprüche

1. Verfahren zur Herstellung von Monocarbonsäuren von Kohlenhydraten, Kohlenhydratderivaten oder primären Alkoholen,
**dadurch gekennzeichnet,**
daß man kontinuierlich Kohlenhydrate, Kohlenhydratderivate oder primäre Alkohole in wäßriger Lösung und Konzentrationen zwischen 0,1 und 60% mit Sauerstoff oder sauerstoffhaltigen Gasen an Edelmetall- oder Mischmetallkatalysatoren oxidiert,
den Volumenstrom der so gebildeten Produkte einer Elektrodialyse zuführt und die Monocarbonsäuren entfernt und gewinnt.

2. Verfahren nach Anspruch 1,
**dadurch gekennzeichnet,**
daß man nicht oxidierte Edukte nach dem Entfernen der Monocarbonsäuren der Oxidationsstufe wieder zuführt.

3. Verfahren nach Anspruch 1 oder 2,
**dadurch gekennzeichnet,**
daß der Stoffstrom vor Betreten des Katalysatorbettes blasenfrei mit Sauerstoff angereichert wird.

4. Verfahren nach Anspruch 3,
**dadurch gekennzeichnet,**
daß in der Oxidationsstufe zunächst ein Reaktor durchlaufen wird, in dem die Sauerstoffanreicherung durch Einleiten von Luft, von Gasgemischen mit höheren Sauerstoffpartialdrücken oder reinem Sauerstoff als feinverteilte Blasen bzw. blasenfrei unter Druck oder drucklos erfolgt, und
daß von dem Reaktor ein Teilstrom durch einen parallel angeordneten Rohrreaktor mit den Katalysatoren gepumpt wird.

5. Verfahren nach Anspruch 3,
**dadurch gekennzeichnet,**
daß in der Oxidationsstufe ein Slurryverfahren mit suspendierten Katalysatoren in einem Rührkessel stattfindet.

6. Verfahren nach Anspruch 5,
**dadurch gekennzeichnet,**
daß die Oxidationsstufe und die Elektrodialysestufe über eine Separationseinrichtung getrennt sind.

7. Verfahren nach einem der vorstehenden Ansprüche,
**dadurch gekennzeichnet,**
daß als Katalysatoren Edelmetalle der 8. Nebengruppe auf Trägern eingesetzt werden.

8. Verfahren nach Anspruch 7,
**dadurch gekennzeichnet,**
daß insbesondere Pt-Träger-Katalysatoren mit Platingehalten zwischen 0,1 bis 10%, insbesondere Pt/C-Pulverkatalysatoren mit Pt-gehalten zwischen 0,1 und 10% mit entferntem feinkörnigem Anteil eingesetzt werden.

9. Verfahren nach einem der vorstehenden Ansprüche,
**dadurch gekennzeichnet,**
daß als Ausgangssubstanzen reduzierende Saccharide, wie z.B. Palatinose, Glucose, Fructose, Sorbose, und/oder nichtreduzierende Saccharide, wie z.B. Saccharose, Trehalose, und/oder Zuckeralkohole wie z.B. Palatinit, Sorbit, und/oder Alkylglycoside sowie Alkylpolyglycoside, wie z.B. Methylglycosid, Octylglycosid bzw. ähnliche Mischungen und/oder speziell modifizierte Kohlenhydratderivate, wie z.B. HMF oder GMF, eingesetzt und monooxidiert werden.

10. Verfahren nach einem der vorherstehenden Ansprüche,
**dadurch gekennzeichnet,**
daß als Lösungsmittel für die Ausgangssubstanzen Wasser oder Mischungen aus Wasser und sekundären Alkoholen, vorzugsweise Isopropanol, eingesetzt werden.

11. Verfahren nach einem der vorstehenden Ansprüche, bei dem die Oxidation und die Elektrodialyse im pH-Bereich von 1 bis 13 erfolgt.

12. Verfahren nach einem der vorstehenden Ansprüche,
**dadurch gekennzeichnet,**
daß die Temperatur für die Oxidation zwischen 0 und 80°C, vorzugsweise zwischen 20 und 60°C liegt.

13. Verfahren nach einem der vorstehenden Ansprüche,
**dadurch gekennzeichnet,**
daß man die Edukte in Konzentrationen zwischen 3 und 20% einsetzt.

14. Verfahren nach einem der vorstehenden Ansprüche,
**dadurch gekennzeichnet,**
daß zur pH-Einstellung Na₂CO₃ oder NaHCO₃ oder NaOH eingesetzt werden oder andere Alkalisierungsmittel.

15. Verfahren nach einem der vorstehenden Ansprüche,
**dadurch gekennzeichnet,**
daß durch Einsatz eines Pt/Al₂O₃-Trägerkatalysators selektiv nur die 6-Position an nicht reduzierenden Glycopyranosyl-Einheiten oxidiert werden.

16. Verfahren nach einem der vorstehenden Ansprüche,
**dadurch gekennzeichnet,**
daß eine Zweigleitung (60) eingesetzt und der von der Oxidationsstufe kommende Stoffmengenstrom vom Stoffmengenstrom der Elektrodialyse entkoppelt wird.

17. Vorrichtung zur Durchführung eines Verfahrens nach einem der vorstehenden Ansprüche 1 bis 16,
**dadurch gekennzeichnet,**
daß sie in Reihe geschaltet eine Begasungsstufe (10), eine Oxidationsstufe (30) und eine Elektrodialysestufe (40) aufweist.

18. Vorrichtung nach Anspruch 17,
**dadurch gekennzeichnet,**
daß zusätzlich eine Leitung (41) zu einem Behälter (50) und eine Rückleitung (43) zur Begasungsstufe (10) vorgesehen ist.

19. Vorrichtung nach Anspruch 17 oder 18,
**dadurch gekennzeichnet,**
daß die Begasungsstufe (10) ein Rührkessel ist.

20. Vorrichtung nach einem der Ansprüche 17 bis 19,
**dadurch gekennzeichnet,**
daß in der Elektrodialysestufe (40) lonenaustauscher- oder bipolare Membranen eingesetzt werden.

21. Vorrichtung nach einem der Ansprüche 17 bis 20,
**dadurch gekennzeichnet,**
daß parallel zur Elektrodialysestufe (40) eine Zweigleitung (60) vorgesehen ist.

## Claims

1. Method for manufacturing monocarboxylic acids from carbohydrates, carbohydrate derivatives or primary alcohols,
**characterised in that**
carbohydrates, carbohydrate derivatives or primary alcohols are oxidized continuously in aqueous solution and concentrations between 0.1 and 60% with oxygen or oxygen-containing gases on noble metal or mixed metal catalysts,
the volume flow of the products so formed is fed to an electrodialysis stage and the monocarboxylic acids are removed and obtained.

2. Method according to claim 1,
**characterised in that**
non-oxidized educts are after the removal of the monocarboxylic acids fed to the oxidation stage once again.

3. Method according to claim 1 or 2,
**characterised in that**
the flow of material is prior to entering the catalyst bed enriched bubble-free with oxygen.

4. Method according to claim 3,
**characterised in that**
in the oxidation stage first of all a reactor is passed through, in which oxygen enrichment takes place by the introduction of air, of gaseous mixtures with higher oxygen partial pressures or pure oxygen as finelydistributed bubbles or bubble-free under pressure or pressureless, and
that from the reactor a part flow is pumped through a tubular reactor disposed in parallel together with the catalysts.

5. Method according to claim 3,
**characterised in that**
in the oxidation stage a slurry method with suspended catalysts takes place in an agitator vessel.

6. Method according to claim 5,
**characterised in that**
the oxidation stage and the electrodialysis stage are separated by a separation unit.

7. Method according to any one of the preceding claims,
**characterised in that**
there are used as catalysts noble metals of the 8th sub-group on supports.

8. Method according to claim 7,
**characterised in that**
in particular Pt supported catalysts with platinum contents between 0.1 and 10%, in particular Pt/C powder catalysts with Pt contents between 0.1 and 10% with the fines removed are used.

9. Method according to any one of the preceding claims,
**characterised in that**
reducing saccharides, such as e.g. palatinose, glucose, fructose, sorbose, and/or non-reducing saccharides, such as e.g. saccharose, trehalose, and/or sugar alcohols such as e.g. palatinitol, sorbitol, and/or alkyl glycosides and alkyl polyglycosides, such as e.g. methyl glycoside, octyl glycoside or similar mixtures and/or specially modified carbohydrate derivatives, such as e.g. HMF or GMF, are used as starting substances and monooxidized.

10. Method according to any one of the preceding claims,
**characterised in that**
there are used as solvents for the starting substances water or mixtures of water and secondary alcohols, for preference isopropanol.

11. Method according to any one of the preceding claims, in which the oxidation and the electrodialysis take place in the pH range from 1 to 13.

12. Method according to any one of the preceding claims,
**characterised in that**
the temperature for the oxidation lies between 0 and 80 °C, for preference between 20 and 60 °C.

13. Method according to any one of the preceding claims,
**characterised in that**
the educts are used in concentrations between 3 and 20%.

14. Method according to any one of the preceding claims,
**characterised in that**
Na₂CO₃ or NaHCO₃ or NaOH are used for the pH adjustment or other alkalizing agents.

15. Method according to any one of the preceding claims,
**characterised in that**
by the use of a Pt/Al₂O₃ supported catalyst selectively only the 6-positions on non-reducing glycopyranosyl units are oxidized.

16. Method according to any one of the preceding claims,
**characterised in that**
a branch line (60) is used and the flow of material coming from the oxidation stage is isolated from the flow of material of the electrodialysis stage.

17. Apparatus for carrying out a method according to any one of the preceding claims,
**characterised in that**
it comprises, connected in series, a gassing stage (10), an oxidation stage (30) and an electrodialysis stage (40).

18. Apparatus according to claim 17,
**characterised in that**
in addition a line (41) to a tank (50) and a return line (43) to the gassing stage (10) are provided.

19. Apparatus according to claim 17 or 18,
**characterised in that**
the gassing stage (10) is an agitator vessel.

20. Apparatus according to any one of claims 17 to 19,
**characterised in that**
ion exchange or bipolar membranes are used in the electrodialysis stage (40).

21. Apparatus according to any one of claims 17 to 20,
**characterised in that**
a branch line (60) is provided parallel with the electrodialysis stage (40).

## Revendications

1. Procédé de préparation d'acides monocarboxyliques dérivant d'hydrates de carbone (de glucides), de dérivés de glucides ou d'alcools primaires, caractérisé en ce qu'on oxyde en continu des glucides, des dérivés de glucides ou des alcools primaires en solution aqueuse et en des concentrations comprises entre 0,1 et 60 %, avec de l'oxygène ou avec des gaz contenant de l'oxygène, en opérant sur des catalyseurs à base de métaux nobles ou de mélanges de métaux,
on achemine le courant en volume des produits ainsi formés vers une électrodialyse et l'on sépare et récupère les acides monocarboxyliques.

2. Procédé selon la revendication 1, caractérisé en ce qu'après avoir enlevé les acides monocarboxyliques, on achemine à nouveau vers l'étage d'oxydation les produits de départ non oxydés.

3. Procédé selon la revendication 1 ou 2, caractérisé en ce qu'on enrichit en oxygène, sans bulles, le courant des matières avant l'entrée de ce courant dans le lit du catalyseur.

4. Procédé selon la revendication 3, caractérisé à ce que, dans l'étape d'oxydation, est parcouru un réacteur dans lequel a lieu l'enrichissement en oxygène par introduction d'air, de mélange(s) de gaz à plus forte pression partielle d'oxygène ou d'oxygène pur sous forme de bulles finement réparties ou sans bulles, sous pression ou sans pression, et en ce que, du réacteur, un courant partiel est pompé pour être envoyé dans un réacteur tubulaire disposé en parallèle et comportant le catalyseur.

5. Procédé selon la revendication 3, caractérisé en ce que, dans l'étape d'oxydation, se produit un procédé en suspension ("slurry") avec des catalyseurs en suspension dans un récipient ou une chaudière comportant un dispositif d'agitation.

6. Procédé selon la revendication 5, caractérisé en ce que l'étape d'oxydation et l'étape d'électrodialyse sont séparées par une installation de séparation.

7. Procédé selon l'une des revendications précédentes, caractérisé en ce qu'on utilise comme catalyseurs des métaux nobles du 8ème sous-groupe sur des supports.

8. Procédé selon la revendication 7, caractérisé en ce qu'on utilise notamment des catalyseurs à base de Pt sur un support, comportant des teneurs en platine de 0,1 à 10 %, notamment des catalyseurs pulvérulant Pt/C ayant des teneurs en Pt comprises entre 0,1 et 10 % avec enlèvement de la fraction en grains fins.

9. Procédé selon l'une des revendications précédentes, caractérisé en ce qu'on utilise et soumet à mono-oxydation, comme substances de départ des saccharides réducteurs comme par exemple du palatinose, du glucose, du fructose, du sorbose et/ou des saccharides non réducteurs, comme par exemple le saccharose, le tréhalose et/ou des sucres ou -alcools comme par exemple du palatinitol, du sorbitol et/ou des alkylglycosides ainsi que des alkylpolyglycosides comme par exemple du méthylglycoside, de l'octylglycoside ou des mélanges analogues et/ou des dérivés de glucides spécialement modifiés comme par exemple HMF ou GMF.

10. Procédé selon l'une des revendications précédentes, caractérisé en ce qu'on utilise, comme solvant pour les substances de départ de l'eau ou des mélanges d'eau et d'alcools secondaires, avantageusement l'isopropanol.

11. Procédé selon l'une des revendications précédentes, dans lequel l'oxydation et l'électrodialyse ont lieu dans un intervalle du pH de 1 à 13.

12. Procédé selon l'une des revendications précédentes, caractérisé en ce que la température pour l'oxydation se situe entre 0 et 80°C, avantageusement entre 20 et 60°C.

13. Procédé selon l'une des revendications précédentes, caractérisé en ce qu'on utilise les produits de départ en des concentrations comprises entre 3 et 20 %.

14. Procédé selon l'une des revendications précédentes, caractérisé en ce que, pour ajuster le pH, on utilise Na₂CO₃ ou NaHCO₃ ou NaOH ou l'on utilise d'autres agents d'alcalinisation.

15. Procédé selon l'une des revendications précédentes, caractérisé en ce que, grâce à l'utilisation d'un catalyseur Pt sur Al₂O₃ comme support, on oxyde sélectivement en position 6 seulement des motifs glycopyranosyles non réducteurs.

16. Procédé selon l'une des revendications précédentes, caractérisé en ce qu'on utilise un conduit dérivé ou de ramification (60) et en ce que le courant des matières provenant de l'étape d'oxydation est découplé du courant des matières provenant de l'électrodialyse.

17. Dispositif pour la mise en oeuvre d'un procédé selon l'une des revendications 1 à 16 précédentes, caractérisé en ce qu'il présente un étage de traitement par du gaz (10), un étage d'oxydation (30) et un étage d'électrodialyse (40), tous reliés en série.

18. Dispositif selon la revendication lè, caractérisé en ce qu'il comporte en outre un conduit (41) menant à un récipient (50) et un conduit de recyclage (43) menant vers l'étage (10) de traitement par du gaz.

19. Dispositif selon les revendications 17 ou 18, caractérisé en ce que l'étage de traitement par du gaz (10) est un récipient ou une chaudière comportant un dispositif d'agitation.

20. Dispositif selon l'une des revendications 17 et 19, caractérisé en ce que, dans l'étage d'électrodialyse (40), on utilise des membranes échangeuses d'ions ou des membranes bipolaires.

21. Dispositif selon l'une des revendications 17 à 20, caractérisé en ce qu'on prévoit un conduit de dérivation ou de ramification (60), parallèle à l'étage d'électrodialyse (40).
